# EUROPÄISCHE PATENTSCHRIFT

(11) **EP 0 921 111 B1**
(45) Veröffentlichungstag und Bekanntmachung des Hinweises auf die Patenterteilung: **24.07.2002**
(21) Anmeldenummer: 98122148.4
(22) Anmeldetag: 25.11.1998
(51) Int. Cl.: C07C 46/02, C07C 50/16, C07C 50/18, C07C 50/20, C07C 45/69, C07C 49/683

(54) **Verfahren zur Herstellung von substituierten Anthrachinonen**
Process for the preparation of substituted anthraquinones
Procédé pour la préparation d'anthraquinones substituées

(30) Priorität: 02.12.1997 DE 19753484
(43) Veröffentlichungstag der Anmeldung: 09.06.1999
(73) Patentinhaber: BASF AKTIENGESELLSCHAFT, 67056 Ludwigshafen (DE)
(72) Erfinder: Siegel, Wolfgang, Dr., 67117 Limburgerhof (DE); Kramer, Andreas, Dr., 67251 Freinsheim (DE)
(74) Vertreter: Isenbruck, Günter, Dr.

(56) Entgegenhaltungen:
- DE-A- 2 751 662
- FR-A- 673 825
- FR-A- 1 253 697
- FR-A- 2 111 190
- PATENT ABSTRACTS OF JAPAN vol. 007, no. 056 (C-155), 8. März 1983 & JP 57 206633 A (KAWASAKI KASEI KOGYO KK), 18. Dezember 1982
- PATENT ABSTRACTS OF JAPAN vol. 008, no. 015 (C-206), 21. Januar 1984 & JP 58 180452 A (KAWASAKI KASEI KOGYO KK), 21. Oktober 1983

## Beschreibung

Die Erfindung betrifft ein Verfahren zur Herstellung von substituierten Anthrachinonen.

Es ist bekannt, substituierte 9,10-Anthrachinone ausgehend von 1,4-Naphthochinon und substituierten 1,3-Dienen herzustellen. Dabei wird in einer ersten Synthesestufe 1,4-Naphthochinon mit einem substituierten 1,3-Dien in einer Cycloadditionsreaktion (Diels-Alder-Reaktion) zum substituierten Tetrahydroanthrachinon umgesetzt. In einer zweiten Synthesestufe erfolgt die Oxidation des substituierten Tetrahydroanthrachinons zum substituierten Anthrachinon, wobei die Oxidation auch stufenweise über das substituierte Dihydroanthrachinon erfolgen kann.

In der US 2,072,867 ist die Herstellung von in 2-Stellung mit Alkyl (Methyl, Butyl, Heptyl) bzw. Phenyl substituierten 9,10-Anthrachinonen aus 1,4-Naphthochinon und den entsprechend substituierten Dienen Isopren, 2-Butylbutadien, 2-Heptylbutadien und 2-Phenylbutadien beschrieben. Der Cycloadditionsschritt wird in Ethanol als Lösungsmittel bzw. ohne Verwendung eines zusätzlichen Lösungsmittels bei 90 bis 100°C durchgeführt. Das erhaltene substituierte 1,4,4a,9a-Tetrahydroanthrachinon wird anschließend in einer alkoholischen Kaliumhydroxid-Lösung zum entsprechend substituierten 9,10-Anthrachinon oxidiert.

Das Arbeiten ohne Lösungs- oder Verdünnungsmittel bringt den gravierenden Nachteil mit sich, daß die Reaktionswärme nicht wirksam abgeführt werden kann. Auch kann eine ausreichende Durchmischung der Reaktionskomponenten nicht sichergestellt werden.

Die DE-A-21 50 337 beschreibt die Herstellung von 1-Alkenyl-9,10-Anthrachinonen, die darüber hinaus auch in 2- und 3-Stellung substituiert sein können. Dabei wird von 1,4-Naphthochinon und den entsprechend substituierten 1,3,7-Octatrienen ausgegangen. Dabei soll in einem Lösungsmittel gearbeitet werden, in dem vorzugsweise mindestens ein Reaktionsteilnehmer, bevorzugt beide Reaktionsteilnehmer, löslich sind. Als geeignete Lösungsmittel für die Durchführung des Cycloadditionsschritts werden in der Schrift Ketone wie Aceton und Methylethylketon, Ether wie THF und Dioxan, Alkohole wie Methanol, Ethanol und Isopropanol, Ester wie Ethylacetat und Kohlenwasserstoffe wie Benzol und Cyclohexan genannt. Für die Durchführung des Oxidationsschritts wird als geeignete Lösungsmittel neben den vorstehend aufgezählten auch Wasser, als bevorzugtes Medium wird wässriges Ethanol genannt. Als bevorzugter Temperaturbereich für die Durchführung der Oxidation mit Luftsauerstoff werden 20 bis 50°C angegeben. In den Beispielen wird der Cycloadditionsschritt unter Verwendung von Ethanol bzw. Benzol als Lösungsmittel und der Oxidationsschritt unter Verwendung von wässrigem Ethanol als Lösungsmittel durchgeführt, wobei die Luftoxidation des Zwischenproduktes bei Raumtemperatur bzw. unter Eiskühlung durchgeführt wird.

In der JP 59-51235 ist die Synthese von 2-Isohexenyl-9,10-Anthrachinon aus 1,4-Naphthochinon und Myrcen beschrieben, wobei die Oxidation des Zwischenprodukts mit Luftsauerstoff durchgeführt wird. Sowohl im Cycloadditionsschritt als auch im Oxidationsschritt wird in Ethanol als Lösungsmittel gearbeitet. Nachteilig an der Verwendung von Ethanol bzw. allgemein von Alkoholen als Lösungsmittel ist, daß diese unter den Reaktionsbedingungen gegenüber einer Oxidation durch Sauerstoff nicht vollständig inert sind. Dies kann zur Bildung von Nebenprodukten führen, die aufwendige Reinigungsoperationen erforderlich machen können.

Die Luftoxidation des Tetrahydroanthrachinons in Gegenwart leichtflüchtiger organischer Verbindungen wie niedrigsiedenden Alkoholen birgt ferner das Risiko der Bildung explosionsfähiger Gemische aus den leichtflüchtigen organischen Verbindungen und Sauerstoff in sich. Eine großtechnische Duchführung des Verfahrens scheidet dann aus Sicherheitsgründen aus. Leichtflüchtige organische Verbindungen können die verwendeten Lösungsmittel sein. Leichtflüchtige organische Verbindungen können auch in den Ausgangskomponenten als Nebenbestandteile enthalten sein.

Der Gefahr der Bildung explosionsfähiger Gemische kann einerseits dadurch begegnet werden, daß andere Oxidationsmittel als Sauerstoff zum Einsatz kommen.

In US 5,723,675 ist ein Verfahren zur Herstellung von Anthrachinon aus 1,4-Naphthochinon und Butadien beschrieben, bei dem in DMSO als Oxidations- und Lösungsmittel gearbeitet wird. Bei der Reduktion von DMSO wurden Produkte gebildet, die bei der späteren Verwendung von Anthrachinon bei der H₂O₂-Synthese als Katalysatorgifte wirken können. Auch ist das Verfahren wegen der durchzuführenden Extraktionsschritte aufwendig.

In Bioorg. Med. Chem. Lett. 6 (1996), Seite 1859-1864 ist die Synthese von 2-Isohexenyl-9,10-anthrachinon aus 1,4-Naphthochinon und dem Terpen α-Myrcen in Diethylether als Lösungsmittel in Gegenwart einer Lewis-Säure offenbart. Die Oxidation des Diels-Alder-Adduktes erfolgt in Gegenwart von Oxidationsmitteln wie Ag₂O, MnO₂ und DDQ in Diethylether oder Benzol als Lösungsmittel. Die verwendeten Oxidations- und Lösungsmittel sind teuer, physiologisch nicht unbedenklich und für eine großtechnische Verfahrensführung daher ungeeignet.

Die Gefahr der Bildung explosionsfähiger Gemische kann andererseits dadurch vermieden werden, daß in Wasser als Reaktionsmedium gearbeitet wird. Problematisch ist, daß die Ausgangskomponenten Naphthochinon und Dien sowie Zwischen- und Endprodukte der Reaktion in Wasser nicht ohne weiteres löslich sind.

Aufgabe der Erfindung ist es, ein großtechnisch durchführbares Syntheseverfahren fiir Anthrachinone, ausgehend von 1,4-Naphthochinon und 1,3-Dienen das preiswert und verfahrenstechnisch unaufwendig ist und die Nachteile der aus dem Stand der Technik bekannten Verfahren nicht aufweist, bereitzustellen, insbesondere ein Verfahren bereitzustellen, bei dem die Gefahr der Bildung explosionsfähiger Gasgemische im Oxidationsschritt vermieden wird.

Die Aufgabe wurde erfindungsgemäß gelöst durch ein Verfahren zur Herstellung von Anthrachinonen der allgemeinen Formel I 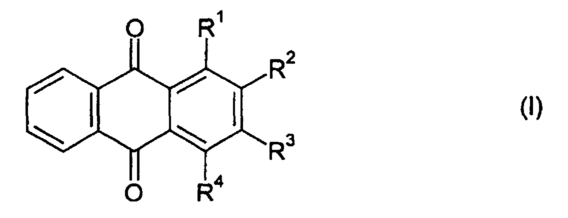 worin R¹, R², R³ und R⁴, die gleich oder verschieden sein können, Wasserstoff, einen C₁-C₈-Alkyl- oder C₂-C₈-Alkenylrest bedeuten, bei dem 1,4-Naphthochinon mit einem 1,3-Dien der allgemeinen Formel II 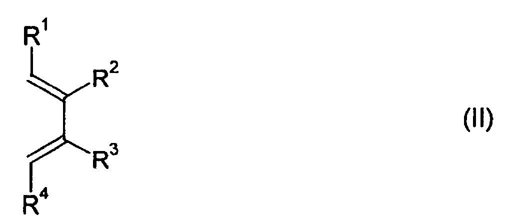 worin R¹, R², R³ und R⁴ die oben angegebene Bedeutung haben, in einem Cycloadditionsschritt zum entsprechenden Tetrahydroanthrachinon umgesetzt wird und das Tetrahydroanthrachinon in einem Oxidationsschritt mit Sauerstoff in Gegenwart eines basischen Katalysators zum Anthrachinon der allgemeinen Formel I umgesetzt wird, das dadurch gekennzeichnet ist, daß sowohl der Cycloadditionsschritt als auch der Oxidationsschritt in Gegenwart von Wasser mit einem Anteil an organischen Lösungsmitteln von < 5 Gew.-% als Verdünnungsmittel durchgeführt werden, wobei vor Durchführung des Oxidationsschnittes leichtflüchtige Nebenbestandteile durch Abdestillieren mit Wasser entfernt werden.

Diene im Sinne dieser Erfindung sind substituierte 1,3-Diene sowie unsubstituiertes 1,3-Butadien. Tetrahydroanthrachinone bzw. Anthrachinone sind demgemäß neben den alkyl- bzw. alkenylsubstituierten Derivaten auch die unsubstituierten Stammverbindungen.

Unter einem wässrigen Verdünnungsmittel im Sinne der Erfindung wird ein Verdünnungsmittel verstanden, das überwiegen aus Wasser besteht. In dem wässrigen Verdünnungsmittel können zu geringen Anteilen mit Wasser mischbare organische Lösungsmittel enthalten sein, wobei der Anteil der organischen Lösungsmittel an dem wäßrigen Verdünnungsmittel < 5 Gew.-% ist. Vorzugsweise ist der Anteil der organischen Lösungsmittel an dem wässrigen Verdünnungsmittel so gering, daß unter den Reaktionsbedingungen der Gleichgewichtsdampfdruck der organischen Lösungsmittel im Gasraum über der Reaktionsmischung gegenüber dem entsprechenden Gleichgewichtsdampfdruck von Wasser so klein ist, daß die Bildung explosionsfähiger Gasgemische ausgeschlossen werden kann. Das ist im allgemeinen der Fall, wenn der Anteil der organischen Lösungsmittel an dem wäßrigen Verdünnungsmittel < 5 Gew.-% ist. In einer besonders bevorzugten Ausführungsform der Erfindung wird ausschließlich mit Wasser als wässrigem Verdünnungsmittel gearbeitet.

In einem ersten Syntheseschritt wird 1,4-Naphthochinon mit einem 1,3-Dien der allgemeinen Formel I zum Tetrahydroanthrachinon umgesetzt. Vorzugsweise liegen die 1,3-Diene unter den Reaktionsbedingungen, insbesonder bei der angestrebten Reaktionstemperatur, bei der die Umsetzung zum Tetrahydroanthrachinon stattfinden, in flüssiger Phase vor. Bevorzugte 1,3-Diene enthalten 4 bis 24 C-Atome, besonders bevorzugt 5 bis 12 C-Atome, speziell bevorzugt 8 bis 10 C-Atome. Beispiele sind Isopren, 2-Ethyl-1,3-butadien, 2-Propyl-1,3-butadien, 2-Allyl-1,3-butadien, 2-(3-Buten-1-yl)-1,3-butadien, 2-(4-Penten-1-yl)-1,3-butadien, 1,3-Pentadien, 1,3-Hexadien, 1,3-Heptadien, 1,3,6-Heptatrien, 1,3,7-Octatrien, 2- bzw. 7-Methyl-, 2- bzw. 7-Ethyl-, 2,7-Dimethyl- bzw. 2,7-Diethyl-1,3,7-octatrien sowie Terpene wie Oxymen und Myrcen. Von diesen ist Myrcen insbesondere bevorzugt.

Das 1,4-Naphthochinon kann ebenfalls Substituenten, beispielsweise Alkylsubstituenten, aufweisen. Bevorzugt wird jedoch unsubstituiertes 1,4-Naphthochinon eingesetzt.

Die Ausgangskomponenten liegen bei Durchführung des Cycloadditionsschrittes in dem wäßrigen Verdünnungsmittel im allgemeinen als Dispersion vor, das heißt als Emulsion, Suspension oder als 3-Phasen-Gemisch (fest/flüssig/flüssig), wobei das wäßrige Verdünnungsmittel geringe Anteile einer oder beider Ausgangskomponenten auch gelöst enthalten kann. Vorzugsweise werden die Ausgangskomponenten so gewählt, daß nach dem Aufheizen auf die Reaktionstemperatur in dem wässrigen Verdünnungsmittel zumindest eine Komponente in flüssiger Form vorliegt. Bei Vorliegen von nur einer Ausgangskomponente in flüssiger Form kann dies das 1,3-Dien oder 1,4-Naphtochinon sein, bevorzugt ist es das 1,3-Dien. Die Reaktionstemperatur bei Durchführung des Cycloadditionsschrittes beträgt im allgemeinen 50 °C bis 170 °C, bevorzugt 70°C bis 130°C, besonders bevorzugt 90 bis 110°C, die Reaktionszeit beträgt im allgemeinen 0,5 bis 24 h, bevorzugt 3 bis 12 h.

In einem zweiten Syntheseschritt wird das gebildete Tetrahydroanthrachinon in Gegenwart von Sauerstoff zum Anthrachinon der allgemeinen Formel I oxidiert. Im allgemeinen liegt das Tetrahydroanthrachinon dabei in dem wäßrigen Verdünnungsmittel dispergiert vor.

Es kann Sauerstoff in reiner Form oder in Gegenwart inerter Gase, zum Beispiel in Form von Luft, eingesetzt werden. Bevorzugt wird als Oxidationsmittel Luftsauerstoff eingesetzt.

Die Oxidation findet in Gegenwart einer in dem wässrigen Verdünnungsmittel löslichen basischen Verbindung statt. Geeignete basische Verbindungen sind Amoniak, Natriumacetat, Mono-, Di- und Trialkylamine, Alkali- und Erdalkalihydroxyde. Bevorzugt findet der Oxidationsschritt in Gegenwart einer stark basischen Verbindung wie Alkali- oder Erdalkalihydroxyd, besonders bevorzugt Alkalihydroxyd statt. Als basische Verbindung wird insbesondere Natriumhydroxyd eingesetzt.

Im Oxidationschritt wird Sauerstoff mit der flüssigen Reaktionsmischung intensiv in Berührung gebracht. Dies kann dadurch geschehen, daß der Luftsauerstoff - vorzugsweise in feinverteilter Form - durch die flüssige Reaktionsmischung geleitet wird. Er kann aber auch dem Gasraum oberhalb der Reaktionmischung zugeführt und durch intensives Rühren mit der Reaktionsmischung in Berührung gebracht werden. Der Oxidationsschritt wird im allgemeinen bei einer Temperatur von 20 bis 170°C, bevorzugt von 50 bis 130°C, besonders bevorzugt von 90°C bis 110°C und während eines Zeitraums von 1 bis 48 h, bevorzugt 4 bis 30 h durchgeführt.

Sowohl der Cycloadditions- als auch der Oxidationsschritt können bei Normaldruck, Unter- oder Überdruck durchgeführt werden. Im allgemeinen wird bei einem Druck von 0,5 bis 30 bar gearbeitet. Das Arbeiten unter Überdruck ist dann angezeigt, wenn bei Temperaturen weit oberhalb des Siedepunktes von Wasser gearbeitet werden soll. Dies kann vorteilhaft sein, wenn hochschmelzende substituierte Diene umgesetzt werden sollen. Arbeiten unter Überdruck ist auch dann angezeigt, wenn ein bei Atmosphärendruck gasförmiges Dien - beispielsweise Butadien - verflüssigt werden soll. In einer bevorzugten Ausführungsform der Erfindung werden Cycloadditions- und Oxidationsschritt bei Normaldruck ausgeführt.

Durch das Arbeiten in Gegenwart eines wässrigen Verdünnungsmittels wird die Gefahr der Bildung explosionsfähiger Gemische vermieden. Dies wird einerseits schon durch den Verzicht auf organische Lösungsmittel erreicht. Explosionsfähige Gasgemische können sich auch aus Sauerstoff und in den Ausgangskomponenten als Nebenbestandteile vorhandenen leichtflüchtigen organischen Verbindungen bilden. Dieser Gefahr kann dadurch begegnet werden, daß im Gasraum über der Reaktionsmischung ein ausreichend hoher Wasserdampfdruck und eine niedrige stationäre Sauerstoffkonzentration eingestellt wird. Wasserdampfdruck und stationäre Sauerstoffkonzentration über der Reaktionsmischung lassen sich bei Erhitzen unter Rückfluß problemlos über die Wärmezufuhr, die die Menge an rücklaufendem Destillat bestimmt, und die Sauerstoffzufuhr einstellen.

Der Gefahr der Bildung explosionsfähiger Gasgemische kann auch dadurch begegnet werden, daß vor Durchführung des Oxidationsschritts leichtflüchtige Nebenbestandteile aus der Raktionsmischung abgetrennt werden. Dies kann bei dem erfindungsgemäßen Verfahren in besonders einfacher Weise durch Andestillieren (teilweises Destillieren) des Reaktionsgemisches geschehen. Beim Andestillieren werden beispielsweise ca. 20 bis 30 Gew.-% des eingesetzten wäßrigen Verdünnungsmittels abdestilliert, wobei leichtflüchtige Nebenbestandteile mit dem Destillat abgetrennt werden. Das abdestillierte wäßrige Verdünnungsmittel kann während des Destillationsvorganges - beispielsweise nach dem Prinzip der Wasserdampfdestillation - ersetzt werden. Die Abtrennung der leichtflüchtigen Nebenbestandteile durch Andestillieren bzw. durch Wasserdampfdestillation ist in Gegenwart eines wäßrigen Verdünnungsmittels problemlos möglich. Die Abtrennung leichflüchtiger Nebenbestandteile kann vor oder nach Durchführung des Cycloadditionsschritts erfolgen. In einer bevorzugten Ausführungsform des erfindungsgemäßen Verfahrens wird sie nach Durchführung des Cycloadditionsschritts durchgeführt.

Nach Durchführung des Oxidationsschritts kann das Anthrachinon der allgemeinen Formel I aus der gebildeten, das Anthrachinon enthaltenden Emulsion abgetrennt werden. Das Anthrachinon kann in fester oder in flüssiger Form abgetrennt werden.

Das Anthrachinon der allgemeinen Formel I kann in fester Form abgetrennt werden. Das Anthrachinon kann durch Kristallisation bei niedriger Temperatur und anschließender Fest/Flüssig-Trennung, beispielsweise durch Zentrifugieren oder Filtrieren, abgetrennt werden. An die Abtrennung können sich übliche Schritte zur Aufreinigung des Kristallisats anschließen. Übliche Aufreinigungsschritte sind beispielsweise das Umkristallisieren aus einem organischen Lösungsmittel. Vor dem Umkristallisieren kann das Kristallisat mit einem geeigneten Lösungsmittel, beispielsweise Wasser, gewaschen werden. Das abgetrennte Kristallisat kann ferne eingeschmolzen und durch Destillation gereinigt werden.

Das Anthrachinon der allgemeinen Formel I kann auch in flüssiger Form abgetrennt werden. Dies kann durch Extraktion der Emulsion mit einem organischen Lösungsmittel erfolgen. Eine Abtrennung kann in flüssiger Form auch ohne Zusatz eines organischen Lösungsmittels erfolgen. In einer bevorzugten Ausführungsform der Erfindung wird die das Anthrachinon enthaltende organische Phase aus der gebildeten Emulsion nach Phasentrennung in flüssiger Form abgetrennt. Aus der abgetrennten organischen Phase kann das Anthrachinon durch übliche Reinigungsoperationen in reiner Form gewonnen werden. Übliche Reinigungsoperationen sind das Extrahieren der abgetrennten organischen Phase mit Wasser, die Destillation der organischen Phase oder die Kristallisation des Anthrachinons aus einem organischen Lösungsmittel. In einer besonders bevorzugten Ausführungsform des erfindungsgemäßen Verfahrens wird die abgetrennte, in flüssiger Form vorliegende organische Phase mit Wasser gewaschen und anschließend destilliert.

Mit dem erfindungsgemäßen Verfahren kann auch ohne Durchführung aufwendiger Reinigungsschritte ein Produkt ausreichender Reinheit erhalten werden. Dies liegt zum einen daran, daß Wasser als Lösungsmittel gegenüber der Oxidation durch Sauerstoff nahezu vollständig inert ist und die Bildung von Verunreinigungen durch Oxidation des Lösungsmittels ausgeschlossen ist. Zum anderen sind in dem erfindungsgemäßen Verfahren gebildeten Nebenprodukte, beispielsweise solche Nebenprodukte, die durch Oxidation von olefinischen Doppelbindungen in den Zwischen- oder Endprodukten gebildet werden, überwiegend wasserlöslich. Dadurch reichem sich diese Nebenprodukte in der wässrigen Phase an und werden nach Durchführung des Oxidationsschritts weitgehend mit der wässrigen Phase abgetrennt. Die Verwendung eines wäßrigen Verdünnungsmittel ermöglicht im übrigen eine problemlose Abtrennung der gebildeten Anthrachinone der allgemeinen Formel I nach Phasentrennung der wässrigen von der das Anthrachinon enthaltenden flüssigen organischen Phase.

In einer besonders bevorzugten Variante wird das erfindungsgemäße Verfahren mit folgenden Schritten durchgeführt:
a) 1,4-Naphthochinon, das Dien der allgemeinen Formel II und das wässrige Verdünnungsmittel werden in einen beheizbaren Reaktionsbehälter, beispielsweise einen Rührkessel, eingeleitet.
b) 1,4-Naphthochinon und das Dien der allgemeinen Formel II werden in dem wässrigen Verdünnungsmittel, beispielsweise durch Rühren, dispergiert. Einleiten der Ausgangskomponenten und Dispergieren können gegebenenfalls unter Schutzgasatmosphäre durchgeführt werden.
c) Die Dispersion, die feste Bestandteile enthalten kann, wird unter Schutzgasatmosphäre zur Umsetzung von 1,4-Naphthochinon und des Diens der allgemeinen Formel II auf die Reaktionstemperatur aufgeheizt. Durch das Aufheizen schmilzt das Dien und bildet eine Emulsion in dem wässrigen Verdünnungsmittel.
d) Zur Abtrennung von leichtflüchtigen Nebenbestandteilen wird die gebildete, das Tetrahydroanthrachinon enthaltende Emulsion andestilliert. Die Abtrennung der leichtflüchtigen Nebenbestandteile ist abgeschlossen, wenn ca. 20 bis 30 Gew.-% der eingesetzten Wassermenge übergegangen sind. Diese Wassermenge kann während des Destillationsvorganges, zum Beispiel nach dem Prinzip der Wasserdampfdestillation, ersetzt werden.
e) In den Reaktionsbehälter wird Alkalihydroxyd, beispielsweise durch Einleiten einer wässrigen Natriumhydroxyd-Lösung, gegeben.
f) In den Reaktionsbehälter wird. Luftsauerstoff eingeleitet und mit dem Tetrahydroanthrachinon bei ca. 90°C zur Reaktion gebracht. Die stationäre Sauerstoffkonzentration über der flüssigen Reaktionsmischung wird über die Wärmezufuhr und die Sauerstoffzufuhr derart geregelt, daß die Sauerstoffkonzentration im Gasraum über der Lösung maximal 5 Vol-% beträgt.
g) Nach Durchführung des Oxidationsschritts wird Phasentrennung der das Anthrachinon der allgemeinen Formel I enthaltenden Emulsion in eine flüssige organische Phase und eine wässrige Phase herbeigeführt und die flüssige organische Phase als Rohprodukt abgetrennt. Die Reinhheit des Rohproduktes kann > 97 Gew.-% betragen.
h) Die flüssige organische Phase wird zur Gewinnung des reinen Anthrachinons der allgemeinen Formel I üblichen Aufreinigungsschritten unterzogen. Übliche Aufreinigungsschritte sind insbesondere das Extrahieren der flüssigen organischen Phase mit Wasser. Es kann sich eine Destillation anschließen. Die Aufreinigung kann auch entfallen.

Die Erfindung wird durch die folgenden Beispiele näher erläutert

### BEISPIEL 1

In einem Rührbehälter mit Rückflußkühlung werden 180 l Wasser bei Raumtemperatur vorgelegt und unter Rühren 33,3 kg 1,4-Naphthochinon und 40 kg Myrcen zugefahren. Unter Stickstoffatmosphäre wird auf 98 bis 100°C erhitzt und ca. 3 Stunden bei dieser Temperatur gerührt. Anschließend erfolgt die Abtrennung der leichtflüchtigen Komponenten durch Andestillieren des Reaktionsgemisches. Zur Oxidation des Diels-Alder-Adduktes werden 20 kg einer 25%igen wässrigen NaOH-Lösung in den Rührbehälter gefahren und unter Rückfluß das Reaktionsgemisch mit Luftsauerstoff begast. Die stationäre Sauerstoffkonzentration über dem Reaktionsgemisch wird über die Destillatmenge, die über die Wärmezufuhr eingestellt wird, und die Luftzufuhr geregelt. Nach Ende der Oxidation wird auf Raumtemperatur abgekühlt, das Rohprodukt kristallisiert aus und wird abgetrennt. Die Syntheseausbeute ist > 94 % der Theorie.

### BEISPIEL 2

Es wird wie in Beispiel 1 verfahren, jedoch wird nach der Oxidation die flüssige organische Phase abgetrennt und anschließend mit 1801 Wasser bei 90°C extrahiert. Das bei 90 bis 95°C flüssige Rohprodukt wird anschließend über einen Wischblattverdampfer destilliert. Die Destillationsausbeute ist > 90 %.

## Patentansprüche

1. Verfahren zur Herstellung von Anthrachinonen der allgemeinen Formel I 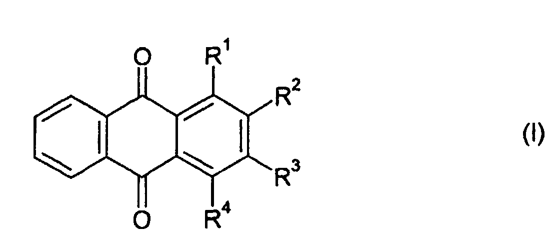 worin R¹, R², R³ und R⁴, die gleich oder verschieden sein können, Wasserstoff, einen C₁-C₈-Alkyl- oder C₂-C₈-Alkenylrest bedeuten, bei dem 1,4-Naphthochinon mit einem 1,3-Dien der allgemeinen Formel II 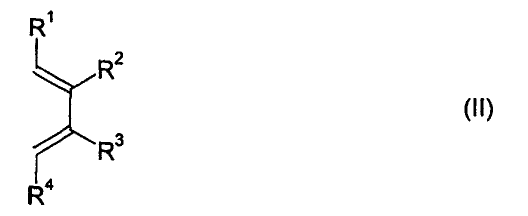 worin R¹, R², R³ und R⁴ die oben angegebene Bedeutung haben, in einem Cycloadditionsschritt zum entsprechenden Tetrahydroanthrachinon umgesetzt werden und das Tetrahydroanthrachinon in einem Oxidationsschritt mit Sauerstoff in Gegenwart eines basischen Katalysators zum entsprechenden Anthrachinon der allgemeinen Formel I umgesetzt wird, **dadurch gekennzeichnet, daß** sowohl der Cycloadditionsschritt als auch der Oxidationsschritt in Gegenwart von Wasser mit einem Anteil an organischen Lösungsmitteln von < 5 Gew.-% als Verdünnungsmittel durchgeführt werden, wobei vor Durchführung des Oxidationsschrittes leichtflüchtige Nebenbestandteile durch Abdestillieren mit Wasser entfernt werden.

2. Verfahren nach Anspruch 1, **dadurch gekennzeichnet, daß** Cycloadditionsschritt und Oxidationsschritt bei einer Umsetzungstemperatur zwischen 90 und 110 °C durchgeführt werden.

3. Verfahren nach Anspruch 1 oder 2, **dadurch gekennzeichnet, daß** sowohl der Cycloadditionsschritt als auch der Oxidationsschritt bei Atmosphärendruck durchgeführt werden.

4. Verfahren nach einem der Ansprüche 1 bis 3, **dadurch gekennzeichnet, daß** nach der Durchführung des Oxidationsschritts aus der gebildeten, das substituierte Anthrachinon enthaltenden Emulsion die organische Phase nach Phasentrennung in flüssiger Form abgetrennt wird.

5. Verfahren nach einem der Ansprüche 1 bis 4, **dadurch gekennzeichnet, daß** als basische Verbindung Alkalihydroxyde eingesetzt werden.

6. Verfahren nach einem der Ansprüche 1 bis 5, **dadurch gekennzeichnet, daß** als als 1,3-Dien Myrcen eingesetzt wird.

7. Verfahren nach einem der Ansprüche 4 bis 6 mit den Schritten:
a) Einleiten von 1,4-Naphthochinon, des Diens der allgemeinen Formel II und des wäßrigen Verdünnungsmittel in einen beheizbaren Reaktionsbehälter;
b) Dispergieren von 1,4-Naphthochinon und des Diens der allgemeinen Formel II in dem wäßrigen Verdünnungsmittel;
c) Aufheizen der Dispersion auf die Reaktionstemperatur zur Umsetzung von 1,4-Naphthochinon und des substituierten Diens der allgemeinen Formel II unter Schutzgasatmosphäre;
d) Abtrennung leichtflüchtiger Nebenbestandteile durch Andestillieren (teilweises Destillieren) der gebildeten, das substituierte Tetrahydroanthrachinon enthaltenden Emulsion;
e) Zugabe von Alkalihydroxyd in den Reaktionsbehälter;
f) Einleiten von Luftsauerstoff in den Reaktionsbehälter und Reaktion mit dem Tetrahydroanthrachinon;
g) Phasentrennung der gebildeten, das Endprodukt enthaltende Emulsion in eine flüssige organische Phase und eine wäßrige Phase und Abtrennung der flüssigen organischen Phase;
h) gegebenenfalls übliche Aufreinigung der flüssigen organischen Phase zur Gewinnung des reinen Anthrachinon der allgemeinen Formel I.

## Claims

1. A process for the preparation of anthraquinones of the general formula I 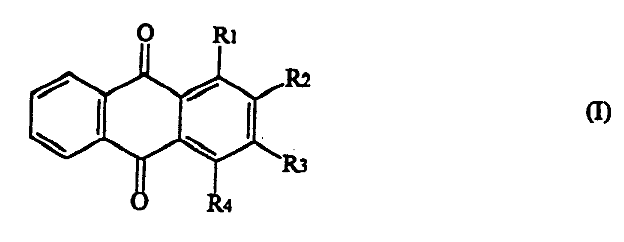 in which R¹, R², R³ and R⁴, which can be identical or different, are hydrogen, a C₁-C₈-alkyl or C₂-C₈-alkenyl radical, in which 1,4-naphthoquinone is reacted with a 1,3-diene of the general formula II 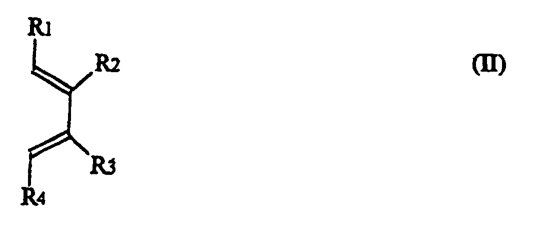 in which R¹, R², R³ and R⁴ have the meaning indicated above, in a cycloaddition step to give the corresponding tetrahydroanthraquinone and the tetrahydroanthraquinone is reacted in an oxidation step with oxygen in the presence of a basic catalyst to give the corresponding anthraquinone of the general formula I, which comprises carrying out both the cycloaddition step and the oxidation step in the presence of water with a proportion of organic solvents of < 5% by weight as diluent readily volatile minor constituents being removed by being distilled off with water, prior to carrying out the oxidation step.

2. The process as claimed in claim 1, wherein the cycloaddition step and oxidation step are carried out at a reaction temperature between 90 and 110°C.

3. The process as claimed in claim 1 or 2, wherein both the cycloaddition step and the oxidation step are carried out at atmospheric pressure.

4. The process as claimed in one of claims 1 to 3, wherein after carrying out the oxidation step the organic phase is removed in liquid form after phase separation from the emulsion formed containing the substituted anthraquinone.

5. The process as claimed in one of claims 1 to 4, wherein the basic compounds employed are alkali metal hydroxides.

6. The process as claimed in one of claims 1 to 5, wherein the 1,3-diene employed is myrcene.

7. The process as claimed in one of claims 4 to 6 having the steps:
a) Introduction of 1,4-naphthoquinone, of the diene of the general formula II and of the aqueous diluent into a heatable reaction container;
b) Dispersion of 1,4-naphthoquinone and of the diene of the general formula II in the aqueous diluent;
c) Heating of the dispersion to the reaction temperature for the reaction of 1,4-naphthoquinone and of the substituted diene of the general formula II under a protective gas atmosphere;
d) Removal of readily volatile minor constituents, by incipient distillation (partial distillation) of the emulsion formed containing the substituted tetrahydroanthraquinone;
e) Addition of alkali metal hydroxide to the reaction container;
f) Introduction of atmospheric oxygen into the reaction container and reaction with the tetrahydroanthraquinone;
g) Phase separation of the emulsion formed containing the final product into a liquid organic phase and an aqueous phase and removal of the liquid organic phase;
h) If desired, customary purification of the liquid organic phase to obtain the pure anthraquinone of the general formula I.

## Revendications

1. Procédé de préparation d'anthraquinones de la formule générale I : 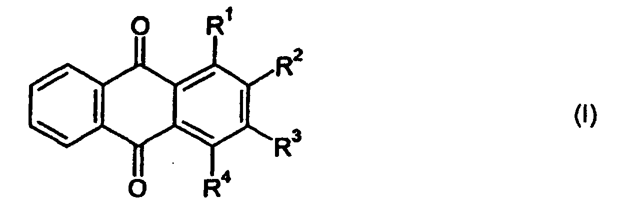 dans laquelle R¹, R², R³ et R⁴, qui peuvent être identiques ou différents, représentent de l'hydrogène, un radical alkyle en C₁-C₈ ou un radical alcényle en C₂-C₈, dans lequel on fait réagir de la 1,4-naphtoquinone avec un 1,3-diène de la formule générale II : 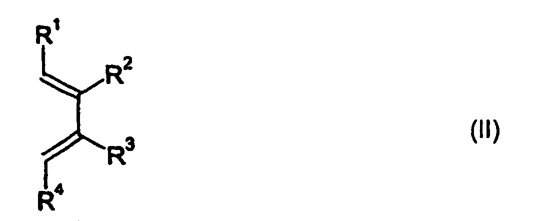 dans laquelle R¹, R², R³ et R⁴ ont la signification indiquée ci-dessus, dans une étape de cycloaddition pour former une tétrahydroanthraquinone correspondante et on fait réagir la tétrahydroanthraquinone dans une étape d'oxydation avec de l'oxygène, en présence d'un catalyseur basique, ce qui donne l'anthraquinone correspondante de la formule générale I, **caractérisé en ce qu'**aussi bien l'étape de cycloaddition que l'étape d'oxydation sont effectuées en présence d'eau avec une fraction de solvants organiques de <5% en poids, comme diluant, des éléments secondaires aisément volatils étant éliminés avec l'eau par séparation par distillation avant d'effectuer l'étape d'oxydation.

2. Procédé suivant la revendication 1, **caractérisé en ce que** l'étape de cycloaddition et l'étape d'oxydation sont effectuées à une température comprise entre 90 et 110°C.

3. Procédé suivant l'une des revendications 1 et 2, **caractérisé en ce qu'**aussi bien l'étape de cycloaddition que l'étape d'oxydation sont effectuées à la pression atmosphérique.

4. Procédé suivant l'une des revendications 1 à 3, **caractérisé en ce que**, après la mise en oeuvre de l'étape d'oxydation, on isole, à partir de l'émulsion formée, contenant l'anthraquinone substituée, la phase organique sous forme liquide après séparation de phases.

5. Procédé suivant l'une des revendications 1 à 4, **caractérisé en ce qu'**on met en oeuvre, comme composé basique, des hydroxydes de métal alcalin.

6. Procédé suivant l'une des revendications 1 à 5, **caractérisé en ce qu'**on met en oeuvre du myrcène comme 1,3-diène.

7. Procédé suivant l'une des revendications 4 à 6, comprenant les étapes :
a) d'introduction de 1,4-naphtoquinone, du diène de la formule générale II et du diluant aqueux dans un récipient réactionnel chauffable,
b) de dispersion de la 1,4-naphtoquinone et du diène de la formule générale II dans le diluant aqueux,
c) de chauffage de la dispersion à la température réactionnelle pour faire réagir la 1,4-naphtoquinone et le diène substitué de la formule générale II sous une atmosphère de gaz de protection,
d) d'isolement des éléments secondaires aisément volatils par une séparation par distillation (distillation partielle) de l'émulsion formée, contenant la tétrahydroanthraquinone substituée,
e) d'addition d'hydroxyde de métal alcalin dans le récipient réactionnel,
f) d'introduction d'oxygène de l'air dans le récipient réactionnel et de réaction avec la tétrahydroanthraquinone,
g) de séparation de phases, dans l'émulsion formée, contenant le produit final, en une phase organique liquide et en une phase aqueuse et d'isolement de la phase organique liquide,
h) éventuellement de purification courante de la phase organique liquide pour la production de l'anthraquinone pure de la formule générale I.
